# EUROPEAN PATENT SPECIFICATION

(11) **EP 1 050 578 B1**
(45) Date of publication and mention of the grant of the patent: **29.05.2002**
(21) Application number: 00113775.1
(22) Date of filing: 23.08.1996
(51) Int. Cl.: C12M 1/00, C12M 3/00, C12M 3/04, C08J 9/26

(54) **Apparatus and methods for culturing biological material**
Verfahren und Vorrichtung zur Kultivierung von biologischem Material
Appareil et procédés de culture de materiau biologique

(30) Priority: 31.08.1995 GB 9517717; 20.12.1995 GB 9526032
(43) Date of publication of application: 08.11.2000
(62) Divisional of application: 96928558.4
(73) Proprietor: Ashby Scientific Ltd., Coalville, Leicestershire LE67 3HF (GB)
(72) Inventor: Fuller, Jess Paul, Hugglescote, Leicestershire LE67 2FP (GB)
(74) Representative: Hughes, Siân Victoria

(56) References cited:
- WO-A-95/24464
- DE-A- 4 132 379
- DE-A- 4 410 718
- FR-A- 2 522 014
- GB-A- 1 092 634
- GB-A- 2 262 538
- US-A- 4 284 275
- US-A- 4 320 198
- US-A- 4 376 826
- US-A- 4 859 712
- US-A- 4 912 058
- DATABASE WPI Section Ch, Week 7637 Derwent Publications Ltd., London, GB; Class A85, AN 76-69743X XP002019186 & JP 51 087578 A (MATSUSHITA ELEC WOR), 1 August 1976 (1976-08-01)
- DATABASE WPI Section Ch, Week 8911 Derwent Publications Ltd., London, GB; Class A35, AN 89-079526 XP002019187 & JP 01 022934 A (POLYPLASTICS KK), 25 January 1989 (1989-01-25)

## Description

The present invention relates to vessels having textured surfaces for growing animal cells, plant cells, micro-organisms and the like ('bio-substances') and to methods for their production.

Many bio-substances can be propagated *in vitro* in containers such as so-called 'roller bottles', that is, cylindrical vessels, usually of glass or polystyrene, which are partially filled with a liquid medium comprising the bio-substances. The bottles are arranged to be rotated slowly about their longitudinal axes, promoting aeration, while the liquid medium provides nutrients to the bio-substances as they grow on the inner surfaces of the container.

Limitations in the efficiency of conventional procedures have hitherto been due to the relatively small surface area provided by the smooth interior of the container, as well as the fact that such surfaces do not provide optimum characteristics for the growth processes. It is an object of the present invention to eliminate or minimise these disadvantages.

Accordingly, in a first aspect of the invention, there is provided apparatus for use in a method of culturing micro-biological material, wherein said apparatus has a rough or uneven growth surface with a plurality of craters or crater-like depressions defined in the inner surface of the apparatus per se or in a coating of an organic polymer formed on the inner surface of the apparatus and, arranged for contact with micro-biological material being cultured. An advantage of the inventive apparatus is that its use can allow cultured material to be grown at enhanced rates.

Preferably, the growth surface is rendered rough or uneven by a plurality of craters or crater like depressions, preferably to depths of 1mm or less and more preferably to depths of 0.1-0.5 mm. Preferably, the craters or crater like depressions measure less than 2mm, and more preferably less than 1mm across. Most preferably, the crater or crater like depressions measure less than 0.5mm across.

In further embodiments, the growth surface is micro-cupellated. The organic polymer coated on the inner surface of the apparatus is preferably a silicone rubber.

In a second aspect, the invention provides a method of culturing a micro-biological material, comprising culturing said material using apparatus in accordance with its first aspect. Advantageously the inventive method allows biological material to be grown at an enhanced rate.

In a third aspect, the invention provides a method of providing an apparatus with a rough or uneven growth surface for culturing microbiological material comprising the steps of forming a mobile surface studded with a granular material on a surface of the apparatus, setting the mobile surface and removing the granular material to leave a rough or uneven surface.

In a fourth aspect of the invention, there is provided an article having a textured surface that can be obtained by a method according to the invention in its third aspect. Preferably, the article is a tissue or cell culture vessel, such as a roller-bottle.

In all aspects of the invention the biological material preferably comprises tissue, cells or like matter, including viral particles or virions.

In the invention according to its fourth aspect, the mobile surface is preferably tacky prior to the setting step, so as to promote adherence of the granular material. In a preferred embodiment, a (preferably solvent-less) silicone paint, designed for polymerisation to form silicone rubber, is applied to the interior surface of a glass or polystyrene roller bottle. While the coating is still in liquid form, common salt particles are injected into the container, for example, under pressure, to adhere to the prepared silicone coating, with any excess salt being removed again from the container. The coating is then allowed to polymerise into a solid layer at ambient or elevated temperature, depending on the type of silicone used. When hardened, the salt particles adhering to the coating are removed by dissolution in water, to leave a surface-porous layer exhibiting a cratered or micro-cupellated structure, as well as the special affinity for bio-substances of silicone rubber. The container so prepared is found to result in a greatly increased yield when used for growth processes in the manner described.

To promote adhesion of the silicone rubber layer within a glass or polystyrene container, a conventional adhesive, preferably a mineral spirit based primer, can be first applied to the surface prior to deposition of the silicone layer.

In embodiments of the invention, it is a pre-requisite that the growth surface should be non-toxic and inert in respect of bio-substances and liquid media. The method of creating such surfaces should not involve any materials, which if remaining on the surface as impurities, could have a deleterious effect on the growth process.

In the above described method, various types of particles can be used in place of common salt, such as, for example, sugar, saltpetre and the like; however, common salt, sucrose or potassium nitrate are preferred as being non-toxic and inert in respect of normal media and bio-substances, as well as being soluble in water for subsequent removal. Solubility of the particles in a non-toxic solvent is an important factor in their choice.

Attempts have been made to use 'open-porous' silicone rubbers, such as the material 'Immobasil' ('Immobasil' is a registered trade mark); however, the results obtained were not as satisfactory as those achievable using the various aspects of the present invention. The open-porous structure (that is, the presence of inter-connected pores within the body of the silicone rubber) in such known materials leads to difficulties in the subsequent harvesting of the biosubstances from the interstices of the coating on completion of a growth cycle.

In an embodiment of the invention, surface pitting or cratering, for example, micro-cupels, can be produced in the inner surfaces of glass or polystyrene containers *per se*. For this purpose, salt or other suitable particles are injected, for example, pneumatically, into the container while the latter is at elevated temperature, either during its manufacture or on subsequent heating. Upon cooling, the particles are then removed as described above, leaving the interior surface of the glass or polystyrene container with a textured, cratered or micro-cupellated structure, again resulting in enhanced efficiency of the process. In the case of polystyrene containers, prior to use, the standard procedure of plasma treatment of the inner surface can be applied to impart a negative electrical charge to the surface, as required for the growth process. Other types of particle can be used apart from salt, the latter however, being again preferred for the reasons enumerated.

It is evident that other methods lying within the ambit of the invention may also be used to derive surface porous, cratered or micro-cupellated textured surfaces of the nature described for growing of bio-substances. The invention can equally be applied to all suitable containers, such as, for example, flasks, tubes, trays, roller bottles, plastic bags and the like.

In applications where very large area growth surfaces are required, it is conventional to utilise containers which comprise flat plates, for example, of polystyrene, wetted by the liquid medium, and upon which the bio-substances are made to grow. Such plates can also be provided with coatings of silicone rubber having a cratered or micro-cupellated, porous, surface structure according to the invention, in the manner described above, to result in substantially enhanced productivity.

Thus, roller bottles and other containers according to the invention can have a specially textured, cratered or micro-cupellated growth surface, usually an inner surface, suitable for use in the growth of bio-substances as aforesaid. By their nature, these surfaces are more conducive to the adhesion and growth of bio-substances, with resulting improved process efficiency. It is considered that the larger surface areas provided by such growth surfaces is a factor in their enhanced performance.

In order that the invention may be better understood, an example thereof will now be described by way of illustration only and with reference to the accompanying drawings, wherein:
Figure 1 is a schematic front perspective view of a conventional roller bottle;
Figure 2 is a schematic side cross-sectional view of the roller bottle of Figure 1 along the line AA' after partial treatment by the inventive method;
Figure 3 is a schematic side cross-sectional view of the roller bottle of Figure 2 after treatment by the inventive method has been completed; and
Figure 4 is a schematic scrap view from a point on the axis of the roller bottle showing a portion of the growth surface after treatment by the inventive method has been completed.

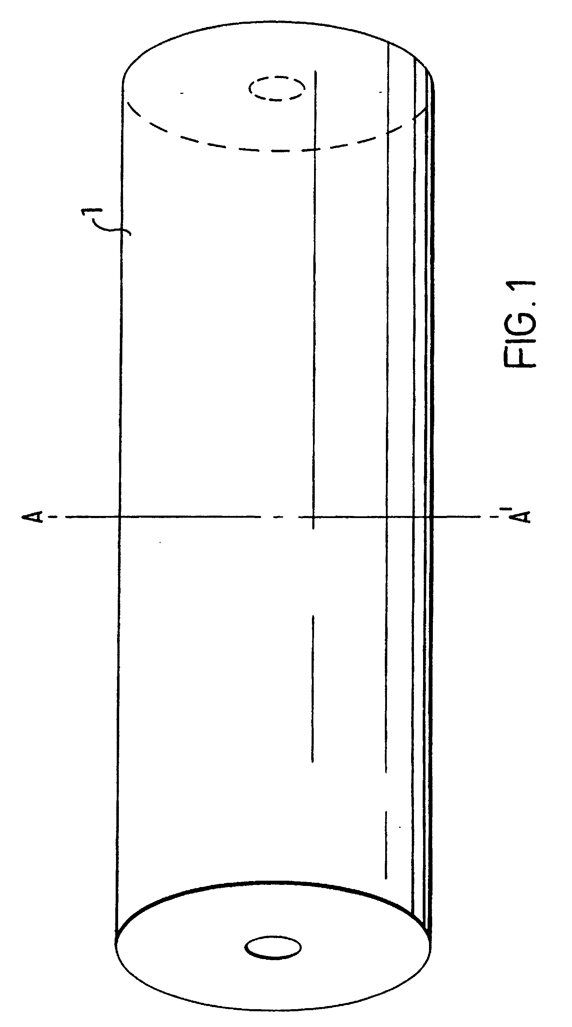

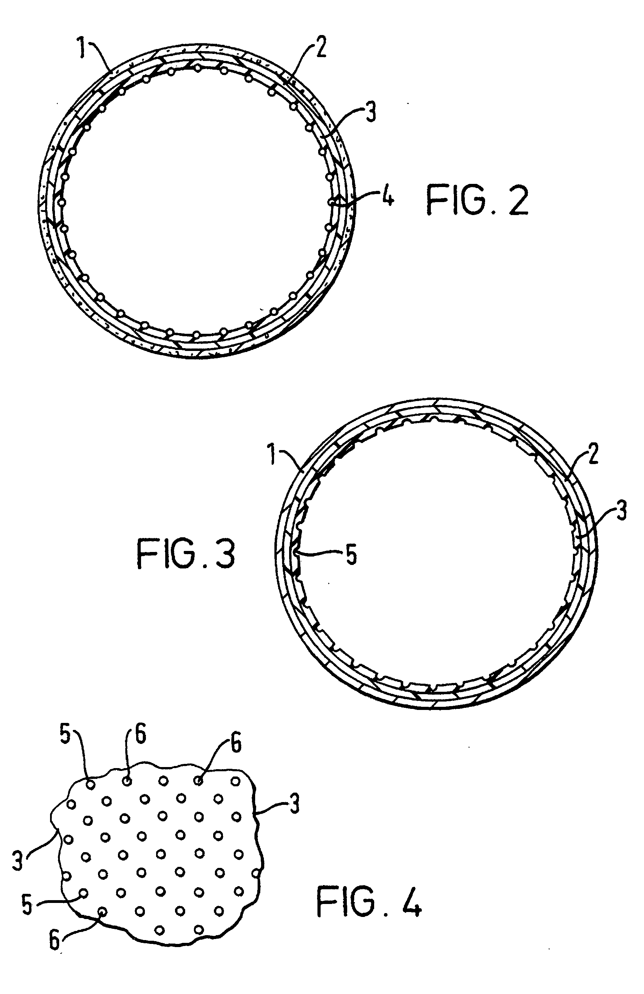

With reference to Figures 1 to 4, a polystyrene roller bottle 1 is provided with a cratered or micro-cupellated surface according to the inventive method as follows. To the interior surface of bottle 1, a coating 2 of silicone rubber primer SS 4155 (General Electric Co., Connecticut, U.S.A.) is applied by brushing or other means and allowed to dry at 70°C for 15 minutes. A coating 3 of silicone rubber paint RTV 118 (General Electric Co., Connecticut, U.S.A.), approximately 0.05 to 0.2mm in thickness, is applied to primer coating 2 and left to stand for 1 minute. A substantial quantity of common salt 4 is then introduced into bottle 1 until approximately 1/3 full and the latter shaken, so that salt 4 adheres to all of the paint coating 3. The excess salt 4 is removed and coating 3 allowed to cure at 70°C for 30 mins. Bottle 1 is then filled with boiling water and allowed to stand for 10 minutes before the water is removed. This last step is repeated once before bottle 1 is rinsed with isopropyl alcohol and left to dry at 70°C for 30 mins. The resultant roller bottle 1 has a textured interior surface 5 provided by craters or micro-cupels 6 inset in silicone rubber layer 4.

The roller bottle 1 can then be inoculated with a biological material, for example cells taken from a commercially available cell line such as Madin Darby Canine Kidney, suspended in an appropriate commercially available nutrient medium, such as Glucose Minimum Essential Medium containing 5% foetal calf serum, selected for its suitability for growing the chosen cells. The roller bottle can then be placed upon conventional equipment designed to gently roll the bottle under conditions conducive to growing cells from the selected line. For example, a common culturing temperature is 37°C. After an appropriate culturing period, the growing cells can be harvested from the roller bottle using conventional techniques, such as trypsination or mechanical scraping.

## Claims

1. Apparatus for use in a method of culturing micro-biological material, wherein said apparatus has a rough or uneven growth surface with a plurality of craters or crater-like depressions defined in the inner surface of the apparatus *per se* or in a coating of an organic polymer formed on the inner surface of the apparatus, and arranged for contact with micro-biological material being cultured.

2. Apparatus as claimed in claim 1, wherein the organic polymer is a silicone rubber.

3. Apparatus as claimed in either of the preceding claims, wherein the crater or crater-like depressions have a depth of 1mm or less and, preferably, a depth of 0.1-0.5mm.

4. Apparatus as claimed in any of the preceding claims, wherein the craters or crater-like depressions measure less than 2mm, preferably less than 1mm, and more preferably less than 0.5mm across.

5. Apparatus as claimed in any of the preceding claims, wherein the growth surface is micro-cupellated.

6. A method of culturing a micro-biological material, comprising culturing said material using apparatus as claimed in any one of the preceding claims, wherein said material is in contact with the rough or uneven growth surface.

7. A method of providing an apparatus with a rough or uneven growth surface for culturing microbiological material, comprising forming a mobile layer studded with a granular material on a surface of the apparatus, setting the mobile layer and removing the granular material to leave a rough or uneven surface.

8. A method as claimed in claim 7, wherein the mobile layer is tacky prior to setting.

9. A method as claimed in claim 7 or 8, wherein the mobile surface is formed by softening the material of the apparatus and studding the softened material with granular material.

10. A method as claimed in claims 7 or 8, wherein the mobile layer is formed by coating the inner surface of the apparatus with a layer of a surface-forming organic polymer and studding said layer with a granular material.

11. A method as claimed in claims 7 or 8, wherein the mobile layer is formed by coating the inner surface of the apparatus with a layer of a surface-forming organic polymer in admixture with a granular material.

12. A method as claimed in claims 10 or 11, wherein the surface-forming organic polymer is a silicone rubber-forming material.

13. A method as claimed in claims 10, 11 or 12, wherein the inner surface of the apparatus is treated with an adhesive prior to coating with the layer of surface-forming organic polymer.

14. A method as claimed in any one of claims 7-13, wherein the apparatus is made of glass or an organic polymer.

15. A method as claimed in any one of claims 7-14, wherein the granular material is soluble and is removed by dissolution.

16. A method as claimed in claim 15, wherein the granular material is sucrose, salt or potassium nitrate.

17. A method as claimed in any one of claims 7-16, wherein the rough or uneven growth surface is cratered or micro-cupellated.

18. An apparatus for use in culturing microbiological material obtainable by a method as claimed in any one of claims 7-17.

## Patentansprüche

1. Vorrichtung zur Verwendung in einem Verfahren zur Kultivierung von mikrobiologischem Material, wobei die genannte Vorrichtung eine rauhe oder unebene Wachstumsoberfläche mit einer Vielzahl von Kratern oder kraterähnlichen Vertiefungen aufweist, die entweder in der Innenwand der Vorrichtung als solcher oder in einer Beschichtung aus einem organischen Polymer eingebracht sind, das auf der Innenwand der Vorrichtung angeordnet ist und das für den Kontakt mit dem zu züchtenden mikrobiologischen Material ausgebildet ist.

2. Vorrichtung gemäß Anspruch 1, wobei das organische Polymer ein Silikongummi ist.

3. Vorrichtung gemäß einem der vorangehenden Ansprüche, wobei die Krater oder kraterähnlichen Vertiefungen eine Tiefe von 1 mm oder weniger und vorzugsweise ein Tiefe von 0.1 - 0.5 mm aufweisen.

4. Vorrichtung gemäß einem der vorangehenden Ansprüche, wobei die Krater oder kraterähnlichen Vertiefungen einen Durchmesser von weniger als 2 mm, vorzugsweise weniger als 1 mm und insbesondere weniger als 0.5 mm aufweisen.

5. Vorrichtung gemäß einem der vorangehenden Ansprüche, wobei die Wachstumsoberfläche mikro-kupelliert ausgebildet ist.

6. Ein Verfahren zum Züchten von mikrobiologischem Material, bei dem das Züchten unter Verwendung einer Vorrichtung gemäß einem der vorangehenden Ansprüche erfolgt, wobei das genannte Material in Kontakt zu der rauhen oder unebenen Wachstumsoberfläche steht.

7. Ein Verfahren zur Herstellung einer Vorrichtung mit einer rauhen oder unebenen Wachstumsoberfläche zum Züchten von mikrobiologischem Material, umfassend die Ausbildung einer beweglichen, mit einem körnigen Material stollenförmig ausgebildeten Oberfläche der Vorrichtung, das Aushärten der beweglichen Schicht und das Entfernen des körnigen Materials, um eine rauhe oder unebene Oberfläche zu erzeugen.

8. Ein Verfahren gemäß Anspruch 7, wobei die bewegliche Schicht vor dem Aushärten klebrig ausgebildet ist.

9. Ein Verfahren gemäß Anspruch 7 oder 8, wobei die bewegliche Oberfläche dadurch gebildet wird, daß das Material der Vorrichtung zunächst erweicht und anschließend das weiche Material mit körnigem Material stollenförmig strukturiert wird.

10. Ein Verfahren gemäß den Ansprüchen 7 oder 8, wobei die bewegliche Schicht gebildet wird, indem die Innenwand der Vorrichtung mit einer Schicht eines oberflächen-bildenden organischen Polymers beschichtet und die genannte Schicht mit einem körnigen Material stollenförmig strukturiert wird.

11. Ein Verfahren gemäß den Ansprüchen 7 oder 8, wobei die bewegliche Schicht durch Beschichten der Innenwand der Vorrichtung mit einer Schicht eines oberflächen-bildenden organischen Polymers gebildet wird, dem ein körniges Material zugemischt wird.

12. Ein Verfahren gemäß den Ansprüchen 10 oder 11, wobei das oberflächen-bildende organische Polymer aus einem gummi-bildenden Silikonmaterial besteht.

13. Ein Verfahren gemäß den Ansprüchen 10, 11 oder 12, wobei die Innenwand der Vorrichtung vor dem Beschichten mit dem oberflächen-bildenden organischen Polymer mit einer klebenden Schicht behandelt wird.

14. Ein Verfahren gemäß einem der Ansprüche 7 - 13, wobei die Vorrichtung aus Glas oder einem organischen Polymer besteht.

15. Ein Verfahren gemäß einem der Ansprüche 7 - 14, wobei das körnige Material löslich ist und mittels Auflösung entfernt wird.

16. Ein Verfahren gemäß Anspruch 15, wobei das körnige Material aus Sucrose, Salz oder Kaliumnitrat besteht.

17. Ein Verfahren gemäß einem der Ansprüche 7 - 16, wobei die rauhe oder unebene Wachstumsoberfläche kraterförmig oder mikro-kupelliert ausgebildet ist.

18. Eine Vorrichtung zur Verwendung beim Züchten mikrobiologischen Materials, die durch ein Verfahren gemäß einem der Ansprüche 7 - 17 herstellbar ist.

## Revendications

1. Appareillage destiné à être utilisé dans une méthode de culture de matériau micro biologique, dans lequel ledit appareillage comporte une surface de croissance rugueuse ou non uniforme présentant une pluralité de cratères ou de creux semblables à des cratères dans la surface interne de l'appareillage en lui-même ou dans un revêtement d'un polymère organique placé sur la surface interne de l'appareillage et destiné à venir en contact avec le matériau micro biologique cultivé.

2. Appareillage selon la revendication 1 dans lequel le polymère organique est un caoutchouc de silicone.

3. Appareillage selon l'une des deux revendications précédentes dans lequel les cratères ou les creux semblables à des cratères ont une profondeur de 1 mm ou moins et de préférence une profondeur de 0,1-0,5 mm.

4. Appareillage selon l'une quelconque des revendications précédentes dans lequel les cratères ou les creux semblables à des cratères ont une dimension transversale inférieure à 2 mm, de préférence inférieure à 1 mm, et plus préférablement inférieure à 0,5 mm.

5. Appareillage selon l'une quelconque des revendications précédentes dans lequel la surface de croissance présente des micro cupules.

6. Une méthode pour la culture d'un matériau micro biologique consistant à cultiver ledit matériau en utilisant un appareillage selon l'une quelconque des revendications précédentes, méthode dans laquelle ledit matériau est mis en contact avec la surface de croissance rugueuse ou non uniforme.

7. Une méthode de création d'un appareillage présentant une surface de croissance rugueuse ou non uniforme en vue de la culture d'un matériau micro biologique, méthode consistant à former une couche mobile parsemée d'une matière granulaire sur une surface de l'appareillage, à fixer la couche mobile et à éliminer la matière granulaire de manière à laisser une surface rugueuse ou non uniforme.

8. Une méthode selon la revendication 7 dans laquelle la couche mobile est collante avant d'être fixée.

9. Une méthode selon l'une des revendications 7 ou 8 dans laquelle la surface mobile est créée en ramollissant la matière de l'appareillage et en parsemant la matière ramollie de matière granulaire.

10. Une méthode selon l'une des revendications 7 ou 8 dans laquelle la couche mobile est formée en revêtant la surface intérieure de l'appareillage avec une couche d'un polymère organique filmogène et en parsemant sur cette couche une matière granulaire.

11. Une méthode selon l'une des revendications 7 ou 8 dans laquelle la couche mobile est formée en revêtant la surface interne de l'appareillage avec une couche d'un polymère organique filmogène en mélange avec une matière granulaire.

12. Une méthode selon l'une des revendications 10 ou 11 dans laquelle le polymère organique filmogène est un matériau de type silicone constituant un caoutchouc.

13. Une méthode selon l'une des revendications 10, 11 ou 12 dans laquelle la surface interne de l'appareillage est traitée par un adhésif avant d'être revêtue par la couche de polymère organique filmogène.

14. Une méthode selon l'une des revendications 7-13 dans laquelle l'appareillage est réalisé en verre ou en un polymère organique.

15. Une méthode selon l'une quelconque des revendications 7-14 dans laquelle la matière granulaire est soluble et est éliminée par dissolution.

16. Une méthode selon la revendication 15 dans laquelle la matière granulaire est du sucrose, du sel ou du nitrate de potassium.

17. Une méthode selon l'une quelconque des revendications 7-16 dans laquelle la surface de croissance rugueuse ou non uniforme présente des cratères ou des micro cupules.

18. Un appareillage destiné à être utilisé pour la culture d'un matériau micro biologique et qui peut être obtenu par une méthode selon l'une quelconque des revendications 7-17.
